# FASCICULE DE BREVET EUROPEEN

(11) **EP 1 515 704 B1**
(45) Date de publication et mention de la délivrance du brevet: **22.04.2009**
(21) Numéro de dépôt: 03760778.5
(22) Date de dépôt: 24.06.2003
(51) Int. Cl.: A61K 9/50, A61K 31/40, A61K 31/401, A61K 38/55

(54) **MICROCAPSULES POUR LA LIBERATION RETARDEE ET CONTROLEE DU PERINDOPRIL**
MIKROKAPSELN FÜR DIE VERZÖGERTE KONTROLLIERTE FREISETZUNG VON PERINDOPRIL
MICROCAPSULES FOR THE DELAYED, CONTROLLED RELEASE OF PERINDOPRIL

(30) Priorité: 24.06.2002 FR 0207778
(43) Date de publication de la demande: 23.03.2005
(73) Titulaire: Les Laboratoires Servier, 92415 Courbevoie Cedex (FR)
(72) Inventeur: HUET DE BAROCHEZ, Bruno, F-45140 Ingre (FR); WUTRICH, Patrick, F-45000 Orleans (FR); LEGRAND, Valérie, F-91330 Yerres (FR); CASTAN, Catherine, F-69530 ORLIENAS (FR); MEYRUEIX, Rémi, F-69009 Lyon (FR)
(86) Numéro de dépôt international: PCT/FR2003/001931
(87) Numéro de publication internationale: WO 2004/000286

(56) Documents cités:
- WO-A-98/56355
- WO-A-99/25374
- GB-A- 2 352 172

## Description

La présente invention concerne des microcapsules permettant la libération retardée et contrôlée du périndopril, ou d'un de ses sels pharmaceutiquement acceptables, destinés à une administration par voie orale.

Plus précisément, l'invention se rapporte à une forme microparticulaires à libération retardée et contrôlée de périndopril ou d'un de ses sels pharmaceutiquement acceptables pour laquelle les phases de libération retardées et contrôlées sont maîtrisées de façon certaine grâce à un double mécanisme : libération "temps dépendant" déclenchée au bout d'une certaine durée de séjour dans l'estomac et libération "pH dépendant" déclenchée par un changement de pH lors de l'entrée des particules dans le petit intestin. Les microparticules de la présente invention sont des microcapsules de granulométrie comprise entre 100 et 1 200 microns contenant du périndopril et individuellement recouvertes par au moins une pellicule d'enrobage permettant la libération retardée et contrôlée de périndopril.

Le périndopril sous forme de sel de tert-butylamine est commercialisé pour le traitement de l'hypertension artérielle et l'insuffisance cardiaque congestive. Il exerce notamment une activité inhibitrice sur certaines enzymes, comme les carboxypolypeptidases, les enképhalinases ou la kininase II. Il inhibe notamment la transformation du décapeptide angiotensine I en l'octapeptide angiotensine II, responsable dans certains cas de l'hypertension artérielle, en agissant sur l'enzyme de conversion.

L'emploi en thérapeutique du périndopril et de ses sels pharmaceutiquement acceptables permet de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Actuellement, le sel de *tert*-butylamine du périndopril est administré par voie orale sous la forme d'un comprimé à libération immédiate.

Il est administré le matin en une prise quotidienne. Or, il est nécessaire pour un meilleur traitement de l'hypertension artérielle non seulement de contrôler la tension artérielle sur l'ensemble du nycthémère mais aussi de s'assurer que le traitement permet de prévenir les remontées tensionnelles observées notamment le matin au lever des patients. Ces remontées tensionnelles dites "pics du petit matin" sont extrêmement difficiles à contrôler et sont responsables de nombreux accidents cardiovasculaires chez des patients hypertendus.

Les comprimés de périndopril actuellement commercialisés permettent une couverture tensionnelle sur l'ensemble du nycthémère mais ne permettent pas de se préserver totalement de la remontée tensionnelle observée au petit matin chez les patients hypertendus. Une étude clinique chez des patients hypertendus a montré qu'avec le comprimé actuel les concentrations plasmatiques en principe actif atteintes entre 4 et 8 heures du matin sont insuffisantes pour que disparaisse complètement cette remontée tensionnelle observée le matin.

Afin de résoudre ce problème, il était nécessaire de mettre au point une nouvelle forme galénique administrable une fois par jour, garantissant la libération et l'absorption du principe actif au moment souhaité et permettant de contrôler efficacement la tension artérielle sur toute la journée et notamment le matin.

Or, force est de constater que la plupart des formes à libération retardée ne peuvent assurer de façon certaine la libération de principe actif dans un délai prescrit.

En effet, de manière conventionnelle, les formes à libération retardée sont obtenues par revêtement du PA par une couche de polymère entérique par exemple de copolymère d'acide méthacrylique et d'ester méthylique d'acide méthacrylique: EUDRAGIT^{®}L. Ce type de revêtement entérique est connu pour présenter une perméabilité réduite dans les conditions de pH acide de l'estomac et se dissoudre lorsque le pH remonte à une valeur proche de celle régnant dans le petit intestin, libérant ainsi le principe actif (PA). Cependant, la variabilité interindividuelle des conditions de pH gastrique et de la durée de la vidange gastrique ne permettant pas d'assurer de façon certaine la libération du PA après une durée déterminée.

Les systèmes à libération retardée purement "temps dépendant" c'est à dire pour lesquels la libération du PA se déclenche au bout d'une durée déterminée de séjour dans le tractus gastro intestinal ne sont pas non plus satisfaisants. En effet, du fait de la variabilité intra et inter individuelle du temps de résidence gastrique, la libération du périndopril peut se produire après que celui ci soit passé devant sa fenêtre d'absorption, qui est localisée pour dans le cas du périndopril, dans la partie haute du tractus gastrointestinal. La bioabsorption peut ainsi être très faible, voire nulle.

Dans ce contexte, il serait particulièrement avantageux de disposer d'une forme galénique à libération retardée et contrôlée du périndopril permettant d'assurer de façon certaine la libération grâce à un double mécanisme de déclenchement de la libération du périndopril : libération "temps dépendant" déclenchée au bout d'une durée contrôlée dans l'estomac et libération "pH dépendant" déclenchée par une remontée du pH lorsque la forme galénique pénètre dans l'intestin. Ces deux facteurs déclencheurs de la libération du périndopril mis en série conféreraient au système galénique une grande sécurité d'emploi. La libération du périndopril serait ainsi garantie après un temps de latence préréglé, même si la variation de pH n'est pas intervenue comme déclencheur.

Il serait aussi avantageux que la forme à libération retardée et contrôlée soit constituée d'une pluralité de microcapsules de diamètre inférieur à 1200 microns. En effet, pour une telle forme, la dose de PA à administrer se répartit entre un grand nombre de microcapsules et présente de ce fait les avantages intrinsèques suivants :
- Le temps de séjour des microcapsules dans les parties hautes du tractus peut être prolongé, ce qui assure un accroissement de la durée de passage du périndopril devant les fenêtres d'absorption et maximise ainsi la biodisponibilité du périndopril.
- La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du PA constant.
- La variabilité de la vidange gastrique est moindre, car elle s'effectue ici sur un grand nombre de particules et est statistiquement plus reproductible.
- On évite la mise en contact des tissus avec une dose élevée en périndopril "dose dumping". Chaque microcapsule ne contient en effet qu'une dose très réduite en périndopril. On s'affranchit ainsi du risque de détérioration des tissus par surconcentration locale de périndopril.
- Il est possible de combiner plusieurs formes galéniques ayant des cinétiques de libération différentes (libération immédiate et/ou retardée et/ou prolongée) comportant plusieurs principes actifs co administrés avec le périndopril, dans ces systèmes "multimicrocapsulaires".
- Il est possible de présenter ces microcapsules sous forme de sachet, gélule ou comprimé.

Enfin, il serait également souhaitable que la couche de revêtement autour des microcapsules soit de faible épaisseur. En effet, une couche de revêtement de forte épaisseur aurait plusieurs conséquences négatives :
(a) la fraction massique en excipient dans la forme galénique serait trop élevée, d'où une masse de médicament trop importante pour être avalée aisément et donc, in fine, des problèmes d'observance qui mettent en péril le succès du traitement ;
(b) le temps de fabrication des microcapsules serait très long, typiquement de plusieurs heures.

Ce problème se pose avec d'autant plus d'acuité pour le périndopril, du fait de sa solubilité très élevée en milieu aqueux.

En définitive, il serait donc particulièrement intéressant de disposer d'une forme galénique à libération retardée et contrôlée de périndopril, ayant simultanément les propriétés suivantes:
- la libération du périndopril peut se déclencher de deux façons : par libération "temps dépendant" lorsque la durée de séjour des particules dans l'estomac excède le temps de latence souhaité avant la libération du périndopril ; ou par libération « pH dépendant » lorsque le système pénètre dans l'intestin. Ces deux facteurs déclencheurs de la libération de périndopril mis en série garantissent la libération du périndopril après un temps de latence préréglé, même si la variation de pH n'est pas intervenue comme déclencheur ;
- elle est constituée d'une pluralité de microcapsules de périndopril enrobées, de petite taille ;
- la fraction massique en excipients d'enrobage est limitée.

La libération retardée ou contrôlée de principes actifs a fait l'objet de nombreux travaux.

La demande FR-A-00 14876 décrit un médicament de traitement des diabètes de type II, comprenant plusieurs milliers de microcapsules d'anti-hyperglycémiques (metformine) constituées chacune par un coeur comportant au moins un anti-hyperglycémique et par une pellicule d'enrobage (e.g acide stéarique et éthylcellulose) appliquée sur le coeur et permettant la libération prolongée in vivo de l'anti-hyperglycémique. Ces microcapsules ont une granulométrie comprise entre 50 et 1000 µm.
Cette demande FR-A-00 14876 n'indique pas comment obtenir la libération retardée et contrôlée de PA, avec un déclenchement "temps dépendant" et "pH dépendant" du PA.

La demande de brevet européen EP-A-0 609 961 divulgue des granulés oraux de morphine, permettant la libération contrôlée du PA s'accélérant par la remontée du pH.
Ces granulés comportent :
o un coeur en sucre (φ= 100 à 1700 µm ),
o enrobé d'une couche d'actif avec un liant (PVP ou HydroxyPropyl-MéthylCellulose : HPMC),
o et une enveloppe extérieure à base :
   ◆ d'un polymère insoluble indépendamment du pH (éthylcellulose ou copolymère d'ester méthacrylique et de méthacrylate d'ammonium: EUDRAGIT® RS ou RL),
   ◆ d'un polymère entérique insoluble à pH acide (copolymère d'acide méthacrylique et d'ester méthylique d'acide méthacrylique: EUDRAGIT® L),
      ◆ d'un composant partiellement soluble à pH acide (polyéthylène glycol, PVP, HPMC, Alcool PolyVinylique : APV),
      ◆ éventuellement un plastifiant (diéthylphtalate),
      ◆ et éventuellement une charge (talc).

Les fractions massiques en PA sont par exemple: 41%, 38,0%, 29,0% ; et les fractions massiques en enveloppe extérieure e.g. : 14,1%, 21,5%, et 12,3% (poids sec).
La libération du PA est présente à tout pH et s'amplifie lorsque le pH passe de pH 1.2 à pH 7.5 . Il s'agit donc d'une forme à libération prolongée et non retardée.

Le brevet US-A-6,033,687 décrit une formulation à base de diltiazem constituée par un mélange de deux types de granulés (φ=1,4 mm) à base de diltiazem : des granulés à temps de latence court et des granulés à temps de latence long. Les profils de libération sont mesurés à pH 1. Ces granulés comprennent :
► un coeur neutre en sucre (φ= 0,5-1,5 mm ),
► une couche de diltiazem associé à un liant (hydroxypropylcellulose, carboxyméthylcellulose, éthylcellulose, polyvinylpyrrofidone, alginate, EUDRAGIT^{®},
► une couche externe unique à base de lubrifiant (talc), de 2 copolymères d'ester méthacrylique et de méthacrylate d'ammonium (EUDRAGIT^{®} RS et EUDRAGIT^{®} RL ; d'un tensioactif (laurylsulfate de sodium) et d'un plastifiant (triéthylcitrate).

Dans les granulés à temps de latence court, la fraction massique de l'enrobage représente 12,3 % contre 30,3 % dans les granulés à temps de latence long. Cette technique ne permet cependant pas d'obtenir des temps de latence longs pour des taux de pelliculage inférieurs à 30 %. Par ailleurs, compte tenu de la variabilité intra et inter individuelle du temps de résidence gastrique, ce système à libération retardée "temps dépendant" peut libérer le PA après que celui ci soit passé devant sa fenêtre d'absorption. Il en résulte une perte importante de biodisponibilité.

Le brevet EP-B-0 263 083 décrit une composition de revêtement de microcapsules permettant d'obtenir un profil de libération de PA d'ordre zéro et reproductible. Cette composition de revêtement est composée d'un mélange :
○ d'un durcisseur polymère assurant la tenue mécanique de cette membrane et qui pouvant être e.g. : éthylcellulose ou copolymère(s) de l'acide méthacrylique (Eudragit E, L S ou RS),
○ d'un composé lipophile, e.g. : acide stéarique ou paraffine,
○ et de talc.
Cette composition de revêtement est présente dans les microcapsules à raison de 15 à 35 % en poids sur sec, par exemple. Les ratios durcisseur polymère/ composé lipophile sont par exemple de 44 et 42 % respectivement dans les exemples 4 et 5.

Les profils obtenus sont des profils sans temps de latence de durée variable. Il n'est ni enseigné, ni mentionné comment obtenir un profil à libération retardée et contrôlée déclenchée au terme du temps de latence et/ou par une variation du pH.

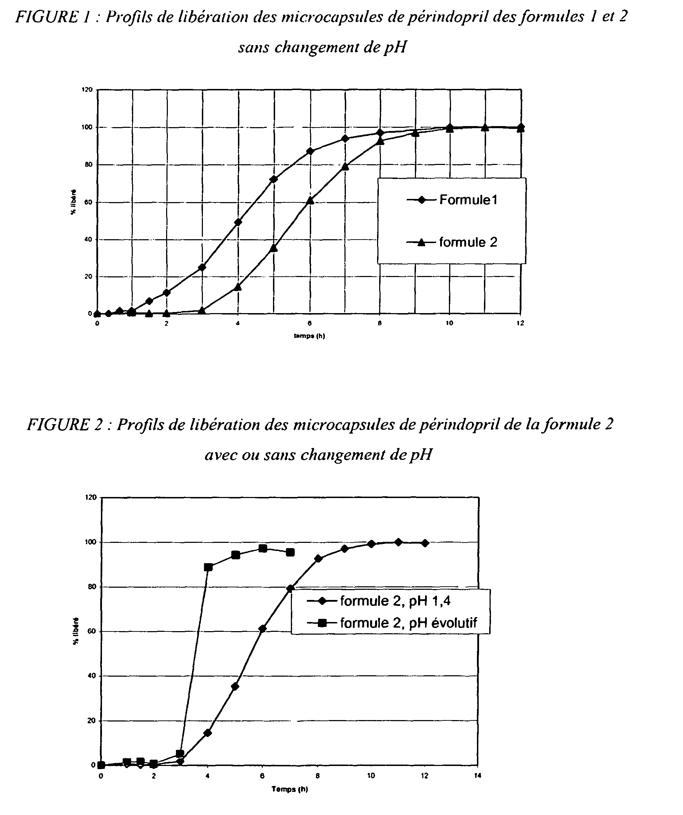

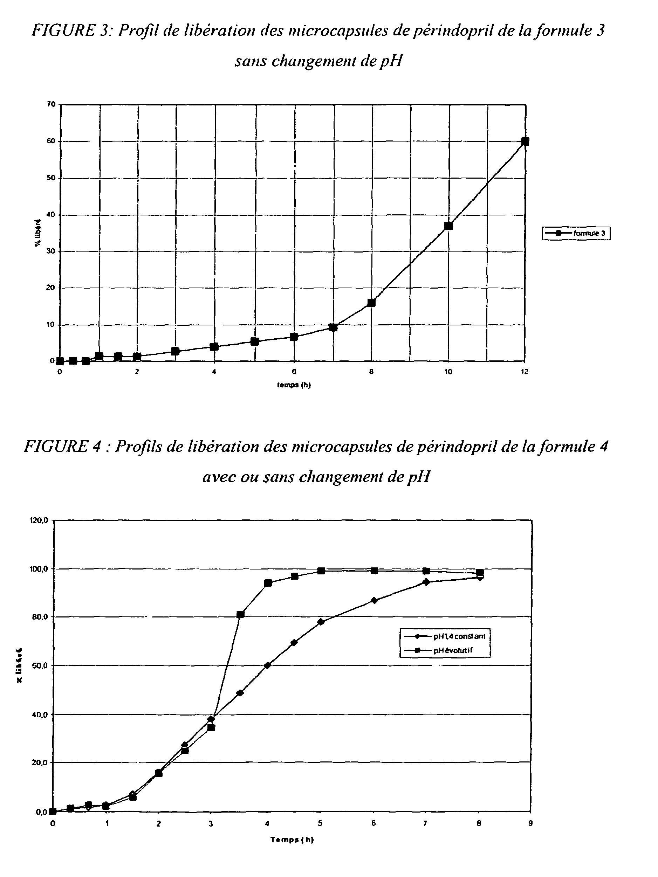

La demande W0 01/58424 A1 divulgue des microcapsules "flottantes" enrobées d'un revêtement entérique par exemple à base d'Eudragit® L, de stéarate de magnésium, de talc et d'un plastifiant tel que le dibutylsébaçate. Ce revêtement peut être enveloppé dans une pellicule "bioadhésive" à base de chitosan e.g. Comme tout revêtement entérique, le revêtement entérique selon ce W0 01/58424, vise une libération "pH dépendant" et non pas la conjonction d'une libération « temps dépendant » et d'une libération "pH dépendant". Par ailleurs, les figures 1 à 3 de cette demande montrent que le simple objectif de libération "pH dépendant" est très imparfaitement atteint puisque jusqu'à 20 % du PA sont libérés en deux heures seulement à pH acide constant. Les particules décrites dans cette demande flottant dans l'estomac, leur temps de séjour gastrique est décrit comme accru, si bien que l'on peut même craindre l'absence de toute libération "pH déclenchée". Finalement, la libération s'effectue de manière incontrôlée par les fuites parasites de PA dans l'estomac.

Dans toutes ces propositions techniques antérieures, la libération du PA s'opère soit sous l'effet de la durée de séjour dans le tractus gastro-intestinal, soit sous l'effet d'une remontée du pH qui intervient lors du passage de l'estomac dans l'intestin grêle. Dans le premier cas, il n'est pas possible d'avoir un temps de latence sans libération de PA (pas de forme retard), et il est à craindre qu'une partie du PA soit libéré in vivo au delà de sa fenêtre d'absorption (parties hautes du tractus gastrointestinal), donc non absorbé dans le cas d'une vidange gastrique trop rapide. Dans le deuxième cas, si la forme galénique stagne dans l'estomac, elle n'est pas soumise à un changement de pH. D'où il s'ensuit que la libération du PA n'intervient pas ou peu. Il va de soi qu'une telle situation est tout à fait fâcheuse, puisque cela équivaut à une absorption du PA trop faible, voire nulle, donc à une inefficacité thérapeutique qui peut s'avérer grave.
Ainsi l'art antérieur ne comprend pas de système galénique permettant de retarder et de garantir de façon certaine la libération d'un principe actif par un double mécanisme de libération "temps dépendant" et de libération "pH dépendant".

D'autre part, il n'existe à ce jour aucune forme à libération retardée et contrôlée d'antihypertenseur de type inhibiteur de l'enzyme de conversion de l'angiotensine.

Dans un tel état de la technique, l'un des objectifs essentiels de la présente invention est de fournir un nouveau système galénique multimicroparticulaire pour l'administration orale de périndopril, ce système étant du type à libération retardée et contrôlée assurant la libération du périndopril de façon certaine grâce à un double mécanisme de libération "temps dépendant" et "pH dépendant". Ces deux facteurs déclencheurs de la libération de périndopril mis en série garantissent la libération du périndopril après un temps de latence préréglé, même si la variation de pH n'est pas intervenue comme déclencheur.

Un objectif essentiel de la présente invention est de proposer une forme galénique formée d'une pluralité de microcapsules permettant de libérer le périndopril à pH 1.4 selon un profil de libération retardée présentant un temps de latence de durée ajustable entre 1 et 8 heures, et de préférence 1 à 5 heures, suivi d'une phase de libération dont le temps de demi libération t_{1/2} est compris entre 0,5 et 25 heures.

Un objectif essentiel de la présente invention est de proposer une fouie galénique formée d'une pluralité de microcapsules permettant de libérer le périndopril selon un profil contrôlé lorsque le pH est passé de 1,4 à 6,8.

Un autre objectif de la présente invention est de proposer une forme galénique constituée d'un grand nombre, par exemple de l'ordre de plusieurs milliers, de microcapsules, cette multiplicité assurant statistiquement une bonne reproductibilité de la cinétique de transit du périndopril dans tout le tractus gastro-intestinal, de sorte qu'il en résulte un meilleur contrôle de la biodisponibilité et donc une meilleure efficacité.

Un objectif essentiel de la présente invention est de proposer une forme galénique de périndopril formée d'une pluralité de microcapsules enrobées évitant l'emploi de fortes quantités d'enrobant.

Un objectif essentiel de la présente invention est de proposer une forme pharmaceutique formée d'une pluralité de microcapsules enrobées permettant de présenter le périndopril sous une forme facile à avaler : sachet, comprimé délitable, gélule, etc...

Un objectif essentiel de la présente invention est de proposer une forme pharmaceutique formée d'une pluralité de microcapsules enrobées permettant de mélanger le périndopril avec plusieurs autres principes actifs .

Un autre objectif de la présente invention est de proposer une forme pharmaceutique formée d'une pluralité de microcapsules enrobées contenant chacune un coeur neutre.

S'étant fixés les objectifs ci-dessus, parmi d'autres, les inventeurs ont eu le mérite de mettre au point, pour assurer une libération certaine et une bonne bioabsorption du périndopril, un système galénique de préférence multimicrocapsulaire ayant pour caractéristique essentielle un double déclenchement de la libération du périndopril. Cela représente un progrès majeur par rapport aux systèmes à libération contrôlée de PA connus jusqu'alors, dans lesquels la libération du PA est déclenchée par un seul facteur : le temps de séjour dans le tractus gastro-intestinal pour certains systèmes, une variation de pH pour d'autres systèmes.

Plus particulièrement, la présente invention concerne des microcapsules "réservoir" pour la libération retardée et contrôlée de périndopril ou d'un de ses sels pharmaceutiquement acceptables destinées à l'administration orale caractérisées en ce que ces microcapsules sont:
◆ constituées par des microparticules de périndopril ou d'un de ses sels pharmaceutiquement acceptables recouvertes chacune d'au moins une pellicule d'enrobage, cette pellicule d'enrobage étant constituée d'un matériau composite comprenant :
   - au moins un polymère hydrophile A porteur de groupements ionisés à pH neutre,
   - au moins un composé hydrophobe B, représentant une fraction massique (% poids par rapport à la masse totale des microcapsules) inférieure ou égale à 40,
◆ et de diamètre inférieur à 1200 microns.

Le polymère hydrophile A porteur de groupements ionisés à pH neutre sera avantageusement choisi parmi les dérivés de la cellulose : cellulose acétate phthalate, hydroxypropyl méthylcellulose phthalate, hydroxypropyl cellulose acétate succinate ; les copolymères d'acide méthacrylique et d'ester d'acide méthacrylique, les copolymères d'acide méthacrylique et d'ester d'acide acrylique (Eudragit^{®} S ou L) et leurs mélanges.

De manière préférentielle, le polymère hydrophile A est un copolymère d'acide méthacrylique et de méthacrylate de méthyle (Eudragit^{®} L 100 / Eudragit^{®} S100) ainsi que le copolymère d'acide méthacrylique et d'acrylate d'éthyle (Eudragit^{®} L100-55).

Le composé hydrophobe B sera avantageusement un composé choisi parmi les cires végétales (Dynasan^{®}P60, Dynasan^{®}P116), les huiles végétales hydrogénées, les triglycérides hydrogénés et leurs mélanges.

De manière préférentielle, le composé hydrophobe B est une huile végétale hydrogénée.

Plus particulièrement, la pellicule d'enrobage de ces microcapsules de périndopril est constituée du mélange de polymère hydrophile A et du composé hydrophobe B dans lequel le ratio pondéral B/A est compris entre 0,2 et 4, de préférence entre 0,5 et 2.

Ce ratio sera ajusté en fonction de la nature des constituants de telle sorte que:
- à pH constant 1.4, le profil de dissolution comporte une phase de latence de durée supérieure ou égale à une demi heure -de préférence comprise entre 1 et 8 heures, et plus préférentiellement encore de 1 à 5 heures,
- le passage, à tout instant pendant la phase de latence, de pH 1.4 à pH 6.8, conduit à une phase de libération du périndopril.

L'un des avantages déterminants du système galénique multimicrocapsulaire, à libération retardée et contrôlée de périndopril, selon l'invention, est de faire intervenir in vivo deux facteurs déclencheurs de la libération du périndopril dans le tractus gastro-intestinal (TGI), à savoir :
- la durée de séjour dans l'estomac : libération "temps déclenchée",
- la variation de pH : libération "pH déclenchée".

Ces deux facteurs déclencheurs de la libération de périndopril sont en série, de sorte qu'ils confèrent au système galénique une grande sécurité d'emploi. La libération du périndopril est ainsi garantie après un temps de latence préréglé, même si la variation de pH n'est pas intervenue comme déclencheur. Les problèmes de variabilité interindividuelle sont ainsi surmontés. L'efficacité du médicament comprenant un tel système galénique est assurée, en respectant une chronobiologie prédéterminée et adaptée à la performance thérapeutique visée.

En outre, pour le périndopril dont la fenêtre d'absorption est limitée, il est particulièrement avantageux que la forme à libération retardée puis contrôlée soit une pluralité de microcapsules "réservoir" et présente de ce fait les avantages intrinsèques suivants :
- Le temps de séjour des microcapsules dans les parties hautes du tractus peut être prolongé, ce qui assure un accroissement de la durée de passage du périndopril devant sa fenêtre d'absorption et maximise ainsi sa biodisponibilité,
- La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du périndopril constant,
- Moindre sensibilité du système à la variabilité de la vidange gastrique, car la vidange qui s'effectue ici sur un grand nombre de particules est statistiquement plus reproductible,
- Possibilité de présenter ces microcapsules sous forme par exemple de sachet, de gélule ou de comprimé.

Le système galénique multimicrocapsulaire selon l'invention permet d'assurer de manière sûre une libération retardée et contrôlée du périndopril dans le TGI, grâce à deux déclencheurs et de se soustraire ainsi à la variabilité inter et intra individuelle des conditions de pH in vivo, lors de la vidange gastrique, tout en étant viable économiquement et facile à ingérer (observance optimisée).

Selon une caractéristique particulièrement avantageuse du mode de réalisation préféré :
■ à pH constant 1,4, la phase de libération contrôlée suivant la phase de latence est telle que le temps de libération de 50 % poids de périndopril (t_{1/2}) se définit comme suit (en heures) : 0,25 ≤ t_{1/2} ≤ 35 de préférence 0,5 ≤ t_{1/2} ≤ 20.

En pratique, la phase de libération du profil de libération in vitro du périndopril à pH 1,4 constant, possède un temps de demi-libération qui est ajustable.

Selon une autre caractéristique intéressante du mode de réalisation préféré :
■ la phase de libération suivant le passage de pH 1,4 à pH 6,8 est telle que le temps de libération de 50 % poids de périndopril (t_{1/2}) se définit comme suit (en heures) : 0,25 ≤ t_{1/2} ≤ 20 de préférence 0,5 ≤ t_{1/2} ≤ 15.

De préférence, les microcapsules selon l'invention comprennent une seule pellicule d'enrobage composite AB. Cela simplifie leur préparation et limite le taux d'enrobage.

Dans les microcapsules selon l'invention, le périndopril sera préférentiellement sous forme de sel de tert-butylamine ou de sel d'arginine.

De préférence, le périndopril est déposé sur un coeur neutre de diamètre compris entre 50 et 600 microns.

Sans que cela ne soit limitatif, il est apparu souhaitable que le coeur neutre soit en sucrose, dextrose, lactose ou cellulose.

Avantageusement, le périndopril est déposé par les techniques connues de l'homme de l'art, par exemple la technique de spray coating en lit d'air fluidisé, sur des coeurs neutres en dextrose ou sucrose de diamètre compris entre 200 et 600 microns.

Sur le plan quantitatif, la monocouche d'enrobant représente au plus 40 %, de préférence au plus 30 % en poids des microcapsules. Un tel taux limité de pelliculage permet de réaliser des unités galéniques contenant chacune une haute dose de principe actif soluble, sans dépasser une taille rédhibitoire au regard de la déglutition. L'observance et donc le succès du traitement ne peuvent que s'en trouver améliorés.

Les microcapsules décrites ci-dessus peuvent être utilisées pour la fabrication de nouvelles compositions pharmaceutiques à base de Périndopril, ayant des performances thérapeutiques optimisées et se présentant de préférence sous forme de comprimés avantageusement délitables et plus préférablement encore orodispersibles, de poudres ou de gélules, et préférentiellement de gélules.

Ces microcapsules sont d'autant plus intéressantes qu'elles sont en outre parfaitement tolérées par l'organisme, notamment au niveau gastrique et par ailleurs peuvent être obtenues de façon aisée et économique.

La présente invention concerne, en outre, ces nouvelles compositions pharmaceutiques en tant que telles, originales dans leur structure, leur présentation et leur composition. Ces compositions pharmaceutiques seront préférentiellement administrées par voie orale, le soir avant le coucher.

Il est à noter qu'il peut être intéressant de mélanger dans une même gélule, un même comprimé ou une même poudre, au moins deux types de microcapsules à cinétiques de libération différentes mais comprises dans le cadre caractéristique de l'invention.

On peut également mélanger les microcapsules selon l'invention avec une certaine quantité de Périndopril immédiatement disponible dans l'organisme.

Il est également envisageable d'associer des microcapsules contenant du Périndopril et des microcapsules contenant des principes actifs différents du Périndopril. A titre préférentiel, des microcapsules d'indapamide pourront être associées aux microcapsules de perindopril.

Ces compositions pharmaceutiques obtenues à partir des microcapsules selon l'invention sont utiles pour le traitement de l'hypertension artérielle et de l'insuffisance cardiaque.

Une étude clinique réalisée chez des patients avec des gélules contenant les microcapsules selon l'invention administrés vers 22 heures a montré que les concentrations plasmatiques en principe actif étaient telles qu'elles permettaient d'atténuer considérablement la remontée tensionnelle observée le matin et d'améliorer le contrôle tensionnel pendant cette période.

D'autre part, il a été montré lors de cette étude clinique qu'avec les microcapsules selon l'invention, la couverture tensionnelle était parfaite sur tout le nycthémère, que le nombre de patients ayant une pression artérielle normalisée était supérieur à celui obtenu avec le comprimé à libération immédiate, et qu'enfin, il y avait une nette amélioration de la variabilité interindividuelle.

Enfin, il a été observé lors de cette étude clinique que contrairement au comprimé à libération immédiate actuellement commercialisé qui doit être pris avant les repas, la prise d'aliments modifiant la biodisponibilité du principe actif, les compositions pharmaceutiques selon l'invention peuvent être administrées avant ou après les repas, sans modification de biodisponibilité.

Les exemples de formules de microcapsules de périndopril ci-dessous illustrent l'invention mais ne la limitent en aucune façon.

### 1- Préparation des microcapsules de périndopril

### Stade A : Préparation de microparticules de périndopril

157 g de sel de tert-butylamine de périndopril et 17 g d'hydroxypropylcellulose sont dispersés ou dissous dans 1300 g d'acetone. La suspension est pulvérisée sur 1500 g de microsphères de sucre de diamètre moyen compris entre 355 et 500 µm dans un spray coater glatt gpcg3. Les conditions de pelliculage sont : température produit : 37-39°c, débit de pulvérisation : 42 g/mn, pression d'atomisation : 1.8 bar.

### Stade B : Préparation des microcapsules de périndopril

Le polymère hydrophile A et le composé hydrophobe B sont dissous dans de l'isopropanol chauffé à une température comprise entre 65 et 75°C. La solution est pulvérisée sur des microparticules de périndopril préparées au stade A dans un spray coater Glatt GPCG3. Les conditions de pelliculage sont : température produit : 36-41°C, débit de pulvérisation : 8-12 g/mn, pression d'atomisation : 1.5 bar.

### 2- Exemples de formulations

Les exemples de formulations sont donnés dans le tableau ci-dessous :

| | *Constituants des microcapsules* | | |
|---|---|---|---|
| | *Microparticules de périndopril (g)* | *Polymère A* *Nature* / *Quantité (g)* | *Composé B* *Nature* / *Quantité (g)* |
| Formule 1 | 700 | Eudragit^{®} L100 37 | Huile de palme hydrogénée 56 |
| Formule 2 | 700 | Eudragit^{®} L100 140 | Huile de palme hydrogénée 93 |
| Formule 3 | 700 | Eudragit^{®} L100 93 | Huile de palme hydrogénée 140 |
| Formule 4 | 700 | Eudragit^{®} L100 105 | Huile de palme hydrogénée 70 |
| Formule 5 | 700 | Eudragit^{®} L100-55 105 | Huile de graines de coton hydrogénée 70 |

| | | | |
|---|---|---|---|
| *Remarques :* les quantités d'isopropanol utilisées pour la préparation des formules 1 à 5 sont respectivement égales à 840 g, 2100 g, 2100 g, 1576 g et 1575 g. | | | |

Les microcapsules des formules 1 à 5 ont été testées dans un dissolutest conforme à la pharmacopée maintenu à 37°C et agité à 100 tours/mn à pH constant ou à pH évolutif.

Les résultats de ces tests sont présentés dans les figures 1 à 5 en annexe.

### Figure 1 : Formules 1 et 2

Ces microcapsules ont été testées dans un milieu HCl à pH 1,4. Les profils de libération obtenus avec ces deux formules sont caractéristiques d'une libération retardée et prolongée. Pour ces deux formules, la phase de libération est déclenchée sans changement de pH après des temps de latence respectivement de 1 et 3 heures. On remarquera que malgré des temps de latence différents, les choix appropriés des rapports Eudragit^{®} L100/Huile de palme ont permis d'obtenir des cinétiques de libération voisines durant la phase de libération.

### Figure 2 : Formule 2

Ces microcapsules ont été testées dans un milieu HCl à pH 1,4 pendant 3 heures puis à pH 6,8 ultérieurement.
Les profils de libération sont caractéristiques d'une libération retardée et prolongée. La phase de libération est déclenchée lors du changement de pH à t = 3 heures. L'examen comparatif des profils de libération à pH 1,4 et à pH évolutif démontre donc que la libération peut être déclenchée par un changement de pH ou sans changement de pH.

### Figure 3 : Formule 3

Ces microcapsules ont été testées dans un milieu HCl pH 1,4. Le profil de libération est caractéristique d'une libération retardée et prolongée. La phase de libération est déclenchée sans changement de pH après un temps de latence de 6 heures.

### Figure 4 : Formule 4

Ces microcapsules ont été testées soit à pH constant (1,4) soit à pH évolutif (1,4 pendant 3 heures puis 6,8). Les profils de libération sont caractéristiques d'une libération retardée et prolongée. On constate qu'a pH évolutif, la phase de libération est doublement déclenchée à t = 1 heure sans changement de pH, puis l'élévation du pH à t = 3 heures déclenchent après changement de pH le deuxième mécanisme de libération.

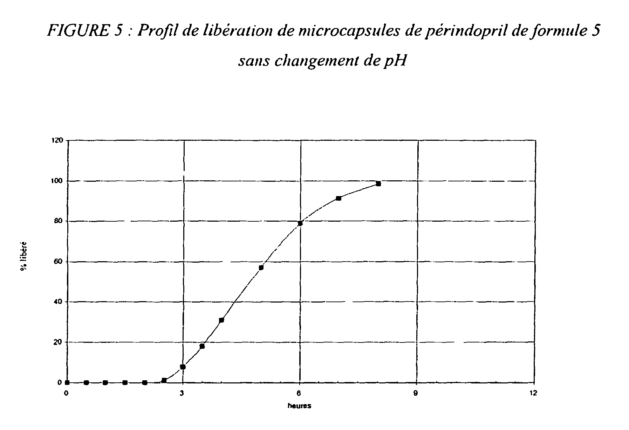

### Figure 5 : Formule 5

Ces microcapsules ont été testées à pH 1,4. Le profil de libération est caractéristique d'une libération retardée et prolongée. La phase de libération est déclenchée sans changement de pH après un temps de latence de 2,5 heures.

## Revendications

1. Microcapsules "réservoir" pour la libération retardée et contrôlée de érindopril ou d'un de ses sels pharmaceutiquement acceptables destinées à l'administration orale **caractérisées en ce que** ces microcapsules sont :
◆ constituées par des microparticules de périndopril ou d'un de ses sels pharmaceutiquement acceptables recouvertes chacune d'au moins une pellicule d'enrobage, cette pellicule d'enrobage étant constituée d'un matériau composite comprenant :
• au moins un polymère hydrophile A porteur de groupements ionisés à pH neutre,
• au moins un composé hydrophobe B, représentant une fraction massique (% poids par rapport à la masse totale des microcapsules) inférieure ou égale à 40,
◆ et de diamètre inférieur à 1200 microns.

2. Microcapsules de périndopril ou d'un de ses sels pharmaceutiquement acceptables selon la revendication 1 **caractérisées en ce que** le polymère hydrophile A est choisi parmi les dérivés de la cellulose, les copolymères d'acide méthacrylique et d'ester d'acide méthacrylique, les copolymères d'acide méthacrylique et d'ester d'acide acrylique et leurs mélanges.

3. Microcapsules selon la revendication 2 **caractérisées en ce que** le polymère hydrophile A est un copolymère d'acide méthacrylique et de méthacrylate de méthyle ou le copolymère d'acide méthacrylique et d'acrylate d'éthyle.

4. Microcapsules selon l'une quelconque des revendications 1, 2 ou 3 **caractérisées en ce que** le composé hydrophobe B est choisi parmi les cires végétales, les huiles végétales hydrogénées, les triglycérides hydrogénés et leurs mélanges.

5. Microcapsules selon l'une quelconque des revendications 1 à 4 **caractérisées en ce que** le composé hydrophobe B est une huile végétale hydrogénée.

6. Microcapsules selon l'une quelconque des revendications 1 à 5 **caractérisées en ce que** la pellicule d'enrobage est constituée du mélange de polymère hydrophile A et du composé hydrophobe B dans lequel le ratio pondéral B/A est compris entre 0,2 et 4.

7. Microcapsules selon l'une quelconque des revendications 1 à 6 telles que la pellicule d'enrobage permette :
- à pH 1,4 d'obtenir un profil de dissolution comportant une phase de latence de durée supérieure ou égale à une demi heure -de préférence comprise entre 1 et 8 heures, et plus préférentiellement encore de 1 à 5 heures,
- d'obtenir une phase de libération du périndopril, à tout instant pendant la phase de latence après passage de pH 1,4 à pH 6,8.

8. Microcapsules selon l'une quelconque des revendications 1 à 7 **caractérisées en ce que** le périndopril est sous forme de sel de tert-butylamine.

9. Microcapsules selon l'une quelconque des revendications 1 à 7 **caractérisées en ce que** le périndopril est sous forme de sel d'arginine.

10. Microcapsules selon l'une quelconque des revendications 8 ou 9 **caractérisées en ce que** le périndopril ou l'un de ses sels pharmaceutiquement acceptable est déposé sur un coeur neutre de diamètre compris entre 50 et 600 microns.

11. Microcapsules selon la revendication 10 **caractérisées en ce que** le coeur neutre hydrophile est en sucrose, dextrose, lactose ou cellulose.

12. Microcapsules selon l'une quelconque des revendications 1 à 11 **caractérisées en ce qu'**elles sont associées à des microcapsules d'indapamide.

13. Utilisation des microcapsules selon l'une quelconque des revendications 1 à 12 pour la préparation de compositions pharmaceutiques de préférence sous forme de comprimés, de poudres ou de gélules.

14. Composition pharmaceutique **caractérisée en ce qu'**elle comprend des microcapsules selon l'une quelconque des revendications 1 à 12.

15. Composition pharmaceutique selon la revendication 14 **caractérisée en ce qu'**elle se présente sous forme de comprimés, de poudres ou de gélules, préférentiellement de gélules.

16. Composition pharmaceutique selon les revendications 14 ou 15 utile pour le traitement de l'hypertension artérielle et l'insuffisance cardiaque.

## Claims

1. "Reservoir" microcapsules for the delayed and controlled release of perindopril or a pharmaceutically acceptable salt thereof for oral administration, **characterised in that** those microcapsules are :
◆ composed of microparticles of perindopril or a pharmaceutically acceptable salt thereof each covered by at least one coating film, that coating film being formed from a composite material comprising :
• at least one hydrophilic polymer A carrying groups ionised at neutral pH,
• at least one hydrophobic compound B, representing a mass fraction (% by weight in relation to the total mass of the microcapsules) less than or equal to 40,
◆ and have a diameter of less than 1200 microns.

2. Microcapsules of perindopril or a pharmaceutically acceptable salt thereof according to claim 1, **characterised in that** the hydrophilic polymer A is selected from cellulose compounds, copolymers of methacrylic acid and a methacrylic acid ester, copolymers of methacrylic acid and an acrylic acid ester and mixtures thereof.

3. Microcapsules according to claim 2, **characterised in that** the hydrophilic polymer A is a copolymer of methacrylic acid and methyl methacrylate or a copolymer of methacrylic acid and ethyl acrylate.

4. Microcapsules according to any one of claims 1, 2 and 3, **characterised in that** the hydrophobic compound B is selected from vegetable waxes, hydrogenated vegetable oils, hydrogenated triglycerides and mixtures thereof.

5. Microcapsules according to any one of claims 1 to 4, **characterised in that** the hydrophobic compound B is a hydrogenated vegetable oil.

6. Microcapsules according to any one of claims 1 to 5, **characterised in that** the coating film is composed of a mixture of hydrophilic polymer A and hydrophobic compound B in which the weight ratio B/A is between 0.2 and 4.

7. Microcapsules according to any one of claims 1 to 6, wherein the coating film enables :
- at a pH of 1.4, a dissolution profile comprising a latent phase of a duration greater than or equal to half an hour - preferably between 1 and 8 hours and more especially from 1 to 5 hours, to be obtained,
- a release phase of perindopril to be obtained at any instant during the latent phase after transition from pH 1.4 to pH 6.8.

8. Microcapsules according to any one of claims 1 to 7, **characterised in that** perindopril is in the form of the tert-butylamine salt.

9. Microcapsules according to any one of claims 1 to 7, **characterised in that** perindopril is in the form of the arginine salt.

10. Microcapsules according to claim 8 or 9, **characterised in that** perindopril or a pharmaceutically acceptable salt thereof is deposited onto a neutral core having a diameter of from 50 to 600 microns.

11. Microcapsules according to claim 10, **characterised in that** the neutral hydrophilic core is made of sucrose, dextrose, lactose or cellulose.

12. Microcapsules according to any one of claims 1 to 11, **characterised in that** they are combined with indapamide microcapsules.

13. Use of the microcapsules according to any one of claims 1 to 12 for the preparation of pharmaceutical compositions preferably in the form of tablets, powders or capsules.

14. Pharmaceutical composition **characterised in that** it comprises microcapsules according to any one of claims 1 to 12.

15. Pharmaceutical composition according to claim 14, **characterised in that** it is presented in the form of tablets, powders or capsules, preferably capsules.

16. Pharmaceutical composition according to claim 14 or 15 for use in the treatment of arterial hypertension and heart failure.

## Patentansprüche

1. "Reservoir"-Mikrokapseln für die verzögerte und gesteuerte Freisetzung von Perindopril oder eines seiner pharmazeutisch annehmbaren Salze für die Verabreichung auf oralem Wege, **dadurch gekennzeichnet, dass** diese Mikrokapseln:
◆ aus Mikroteilchen von Perindopril oder einem seiner pharmazeutisch annehmbaren Salze gebildet sind, die jeweils mit mindestens einer Hüllschicht umgeben sind, welche Hüllschicht aus einem Verbundmaterial gebildet ist, welches:
• mindestens ein ionisierte Gruppen tragendes hydrophiles Polymer A mit neutralem pH-Wert und
• mindestens eine hydrophobe Verbindung B mit einem Massenanteil (Gew.-% bezogen auf die Gesamtmasse der Mikrokapseln) von weniger oder gleich 40 umfasst.
◆ und einen Durchmesser von weniger als 1200 Mikron aufweisen.

2. Mikrokapseln von Perindopril oder einem seiner pharmazeutisch annehmbaren Salze nach Anspruch 1, **dadurch gekennzeichnet, dass** das hydropohile Polymer A ausgewählt ist aus Cellulosederivaten, Copolymeren aus Methacrylsäure und Methacrylsäureestern, Copolymeren aus Methacrylsäure und Acrylsäureestern und Mischungen davon.

3. Mikrokapseln nach Anspruch 2, **dadurch gekennzeichnet, dass** das hydrophile Polymer A ein Copolymer aus Methacrylsäure und Methylmethacrylat oder ein Copolymer aus Methacrylsäure und Ethylacrylat ist.

4. Mikrokapseln nach einem der Ansprüche 1, 2 oder 3, **dadurch gekennzeichnet, dass** die hydrophobe Verbindung B ausgewählt ist aus pflanzlichen Wachsen, hydrierten pflanzlichen Ölen, hydrierten Triglyceriden und Mischungen davon.

5. Mikrokapseln nach einem der Ansprüche 1 bis 4, **dadurch gekennzeichnet, dass** die hydrophobe Verbindung B ein hydriertes pflanzliches Öl ist.

6. Mikrokapseln nach einem der Ansprüche 1 bis 5, **dadurch gekennzeichnet, dass** die Hüllschicht aus einer Mischung des hydrophilen Polymers A und der hydrophoben Verbindung B, bei der das B/A-Gewichtsverhältnis zwischen 0,2 und 4 liegt, gebildet ist.

7. Mikrokapseln nach einem der Ansprüche 1 bis 6, worin die Hüllschicht es ermöglicht:
- bei einem pH-Wert von 1,4 ein Auflösungsprofil zu erhalten mit einer Latenzphase mit einer Dauer von gleich oder mehr als einer halben Stunde - vorzugsweise zwischen 1 und 8 Stunden und noch bevorzugter zwischen 1 bis 5 Stunden.
- eine Phase der Freisetzung von Perindopril zu erzielen zu jedem Zeitpunkt der Latenzphase nach dem Ansteigen des pH-Werts von 1,4 auf einen pH-Wert von 6.8.

8. Mikrokapseln nach einem der Ansprüche 1 bis 7, **dadurch gekennzeichnet, dass** Perindopril in Form des tert.-Butylaminsalzes vorliegt.

9. Mikrokapseln nach einem der Ansprüche 1 bis 7, **dadurch gekennzeichnet, dass** Perindopril in Form des Argininsalzes vorliegt.

10. Mikrokapseln nach einem der Ansprüche 8 oder 9, **dadurch gekennzeichnet, dass** Perindopril oder eines seiner pharmazeutisch annehmbaren Salze auf einem neutralen Kern mit einem Durchmesser zwischen 50 und 600 Mikron abgeschieden ist.

11. Mikrokapseln nach Anspruch 10, **dadurch gekennzeichnet, dass** der hydrophile neutrale Kern aus Saccharose, Dextrose, Lactose oder Cellulose gebildet ist.

12. Mikrokapseln nach einem der Ansprüche 1 bis 11, **dadurch gekennzeichnet, dass** sie mit Indapamid-Mikrokapseln kombiniert sind.

13. Verwendung der Mikrokapseln nach einem der Ansprüche 1 bis 12 für die Herstellung von pharmazeutischen Zubereitungen vorzugsweise in Form von Tabletten, Pulvern oder Gelkapseln.

14. Pharmazeutische Zubereitung, **dadurch gekennzeichnet, dass** sie die Mikrokapseln nach einem der Ansprüche 1 bis 12 umfasst.

15. Pharmazeutische Zubereitung nach Anspruch 14, **dadurch gekennzeichnet, dass** sie in Form von Tabletten, Pulvern oder Gelkapseln, vorzugsweise in Form von Gelkapseln, vorliegt.

16. Pharmazeutische Zubereitung nach den Ansprüchen 14 oder 15 für die Behandlung der arteriellen Hypertension und von Herzinsuffizienz.
